Europäisches Patentamt

(19) European Patent Office

Office européen des brevets

(11) Numéro de publication: **0 252 356**
**B1**

(12) **FASCICULE DE BREVET EUROPEEN**

(45) Date de publication du fascicule du brevet:
03.10.90

(21) Numéro de dépôt: **87108912.4**

(22) Date de dépôt: **22.06.87**

(51) Int. Cl.⁵: **C10G 9/20,** C10G 9/14,
C07C 4/04

(54) Procédé et four de vapocraquage d'hydrocarbures destines à la fabrication d'oléfines et de dioléfines.

(30) Priorité: **25.06.86 FR 8609219**
**25.06.86 FR 8609221**

(43) Date de publication de la demande:
**13.01.88 Bulletin 88/2**

(45) Mention de la délivrance du brevet:
**03.10.90 Bulletin 90/40**

(84) Etats contractants désignés:
**BE DE ES FR GB IT NL SE**

(56) Documents cités:
**DE-B- 1 262 994**
**FR-A- 2 227 314**
**FR-A- 2 512 049**
**GB-A- 1 165 907**
**GB-A- 1 177 181**

(73) Titulaire: **NAPHTACHIMIE S.A., Tour Neptune la**
**Défense 1 20 place de Seine, F-92400 Courbevoie(FR)**

(72) Inventeur: **Martens, André, Les Jardinets Avenue Jules**
**Ferry, F-13220 CHATEAUNEUF LES MARTIGUES(FR)**
Inventeur: **Bellet, Serge, 4, impasse la Fontaine,**
**F-13220 CHATEAUNEUF LES MARTIGUES(FR)**

(74) Mandataire: **Patentanwälte Beetz sen. - Beetz jun.**
**Timpe - Siegfried - Schmitt-Fumian,**
**Steinsdorfstrasse 10, D-8000 München 22(DE)**

## Description

La présente invention se rapporte à un procédé de craquage d'hydrocarbures en présence de vapeur d'eau, destiné à fabriquer des oléfines et dioléfines, et notamment de l'éthylène. La présente invention a également pour objet un dispositif constitué par un four de craquage destiné à la mise en œuvre de ce procédé.

Il est connu de réaliser le craquage à la vapeur d'eau d'hydrocarbures liquides comportant de 5 à 15 atomes de carbone, tels que le naphta, les essences légères et le gas-oil, dans des fours dont la température de sortie est généralement comprises entre 750°C et 850°C, ou d'hydrocarbures gazeux, tels que des alcanes comportant de 2 à 4 atomes de carbone, éventuellement mélangés à du méthane et/ou des alcènes comportant de 2 à 4 atomes de carbone, dans des fours dont la température de sortie est généralement comprise entre 800°C et 880°C. Dans ce procédé, connu sous le nom de craquage ou de pyrolyse à la vapeur d'eau, ou encore sous le nom de vapocraquage, on fait passer à travers la partie radiante d'un four un mélange d'hydrocarbures et de vapeur d'eau circulant dans un tube de craquage disposé sous la forme d'un serpentin à l'intérieur de ce four, la pression de ce mélange à la sortie du four étant généralement comprise entre 120 kPa et 240 kPa. Les hydrocarbures sont ainsi notamment transformés, d'une part, en une fraction hydrocarbonée gazeuse comprenant en particulier des oléfines comportant de 2 à 6 atomes de carbone, telles que l'éthylène, le propylène et l'isobutène, et des dioléfines telles que le butadiène, et, d'autre part, en une fraction hydrocarbonée liquide, dite "gazoline de vapocraquage", comprenant des hydrocarbures comportant de 5 à 12 atomes de carbone, ainsi qu'en sous-produits indésirables, tels que le méthane.

Il est connu, en particulier, que l'éthylène se forme à plus haute température que les oléfines supérieures comportant au moins 3 atomes de carbone. On sait, par ailleurs, que ces oléfines supérieures subissent à des températures élevées en présence d'hydrogène, des réactions secondaires d'hydrocraquage et de condensation, favorisant la formation d'hydrocarbures légers et d'essence. Généralement, dans un tel procédé de vapocraquage, mettant en œuvre des hydrocarbures liquides ou gazeux on détermine le rendement en oléfines, notamment en éthylène, et le rendement en dioléfines, notamment en butadiène, par le rapport pondéral des quantités d'oléfines ou de dioléfines produites à la quantité d'hydrocarbures mise en œuvre. On détermine également le taux pondéral de conversion des hydrocarbures mis en œuvre par l'équation suivante:

taux pondéral de conversion = 100 - (% en poids de la fraction hydrocarbonée à la sortie du four, ayant un intervalle de distillation ASTM 45/205°C).

Les procédés de vapocraquage, connus jusqu'à présent, sont réalisés dans le but d'obtenir un rendement de craquage en éthylène le plus élevé possible. Ainsi, les fours de vapocraquage sont généralement conçus pour fonctionner selon des conditions dites de haute sévérité. Ces conditions sont, en particulier, telles que le mélange d'hydrocarbures et de vapeur d'eau circule en un temps relativement court, généralement inférieur à une seconde, dans un tube de craquage. Par ailleurs, la température de craquage est choisie à une valeur généralement la plus élevée possible, mais qui est limitée par les contraintes thermiques du four et du tube de craquage. Les contraintes thermiques du tube de craquage, liées principalement à sa température de peau, dépendent de la nature du métal ou de l'alliage constituant le tube de craquage. En outre, la pression du mélange circulant dans le tube de craquage est généralement relativement faible, afin de limiter le développement des réactions secondaires. Le four de vapocraquage comprend également un dispositif de chauffe constitué de brûleurs disposés sur la sole et/ou sur les murs internes du four. La puissance thermique de ce dispositif de chauffe est généralement répartie d'une façon homogène le long du tube de craquage, de telle sorte que le mélange d'hydrocarbures liquides et de vapeur d'eau est soumis à une température qui croît rapidement dans la première partie du tube, puis plus lentement dans la deuxième partie du tube. On sait que dans ces conditions le rendement en éthylène d'un four de vapocraquage d'une taille donnée est limité principalement par les contraintes thermiques du tube de craquage et que d'importants inconvénients peuvent apparaître, tels que des phénomènes de cokage à l'intérieur du tube de craquage et un effet de vieillissement prématuré du tube de craquage.

Il a déjà été proposé depuis longtemps, selon le brevet britannique N° 1 177 181, qu'en vue de réduire les phénomènes de cokage, la puissance thermique est appliquée d'une façon non-uniforme le long d'un tube de craquage de section constante, en particulier d'une façon plus intense au début du tube qu'à la fin de celui-ci. Une très légère augmentation du rendement en éthylène a été observée, mais reste cependant limitée à environ 1 %. Par ailleurs, on a constaté que le taux pondéral de conversion des hydrocarbures n'augmente pas dans un tel procédé.

Il est également connu depuis longtemps, par exemple selon le brevet français n° 2 227 314, qu'en vue d'accroître le débit d'hydrocarbures à craquer, les tubes de craquage peuvent être réunis deux par deux pour former ensuite un tube présentant une section transversale proportionnellement plus grande. Ainsi, la section des différents tubes augmente dans la direction de l'écoulement des hydrocarbures à craquer, ce qui permet de transmettre aux hydrocarbures pour une même température, une quantité de chaleur supérieure et d'améliorer ainsi la répartition de la température de peau des tubes de craquage. Cependant, un tel dispositif ne permet pas d'accroître sensiblement le rendement en éthylène et le taux pondéral de conversion des hydrocarbures.

Il est également connu que, compte tenu de la taille considérable des installations industrielles de vapocraquage, il est toujours essentiel d'accroître non seulement le rendement et la production horaire en éthylène d'un four de vapocraquage d'une taille donnée mais aussi le taux pondéral de conversion des hydrocarbures. Cependant, compte tenu du coût élevé des investissements dans ce type d'installation industrielle, il n'est pas concevable économiquement que les modifications envisagées pour atteindre ce but entraînent des transformations trop importantes et coûteuses des installations déjà existantes de vapocraquage. Ainsi, depuis plusieurs années, d'importantes études ont été menées dans ce domaine et des efforts incessants de recherche ont été réalisés aussi bien au stade laboratoire qu'au stade industriel.

Il a été maintenant trouvé un procédé et un dispositif constitué par un four de craquage d'hydrocarbures en présence de vapeur d'eau, permettant d'accroître très sensiblement non seulement le rendement de craquage en éthylène, mais aussi le taux pondéral de conversion des hydrocarbures. Le procédé et le dispositif de l'invention peuvent être, en outre, facilement adaptés aux installations déjà existantes de vapocraquage d'hydrocarbures.

La présente invention concerne tout d'abord un procédé de fabrication d'oléfines et de dioléfines par craquage d'hydrocarbures en présence de vapeur d'eau, consistant à faire passer, à travers une zone de radiation d'un four, un mélange d'hydrocarbures et de vapeur d'eau, circulant dans un tube de craquage placé à l'intérieur de cette zone, en un temps de séjour moyen compris entre 300 et 1800 millisecondes, sous une pression de sortie de four comprise entre 120 kPa et 240 kPa, procédé caractérisé en ce que

(a) la température de craquage du mélange d'hydrocarbures et de vapeur d'eau augmente depuis la température d'entrée de la zone de radiation, comprise entre 500 et 700°C, jusqu'à la température de sortie de cette zone, comprise entre 800 et 880°C, cette augmentation de température étant associée à une répartition non homogène de la puissance thermique du four appliquée le long du tube, répartition telle que la puissance thermique appliquée à la première moitié de la longueur du tube, située vers l'entrée de la zone de radiation, est de 1,5 à 5 fois supérieure à celle appliquée à la deuxième moitié de la longueur du tube, située vers la sortie de cette zone, et

(b) le volume réactionnel de la deuxième moitié de la longuer du tube de craquage, située vers la sortie de la zone de radiation, est de 1,3 à 4 fois supérieur à celui de la première moitié de la longueur du tube, située vers l'entrée de cette zone.

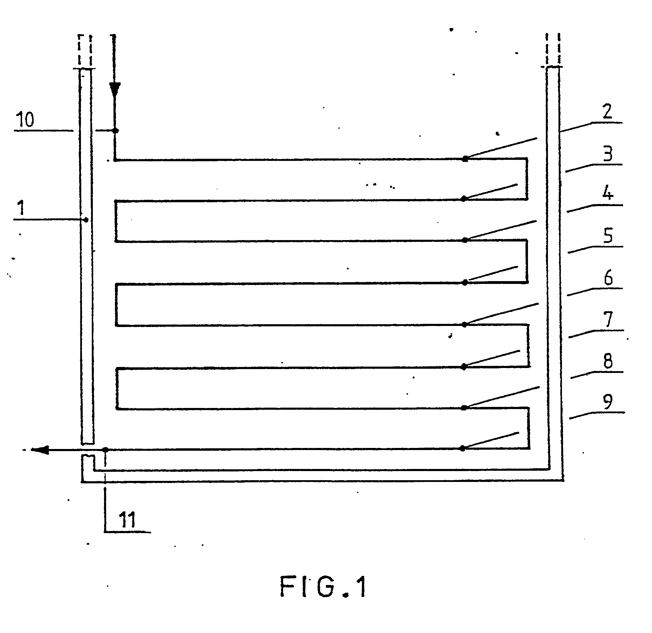

La figure 1 est une illustration schématique d'un four horizontal de vapocraquage, comprenant une enceinte thermique de radiation, encore applée zone de radiation, à travers laquelle passe un tube de craquage disposé sous la forme d'un serpentin.

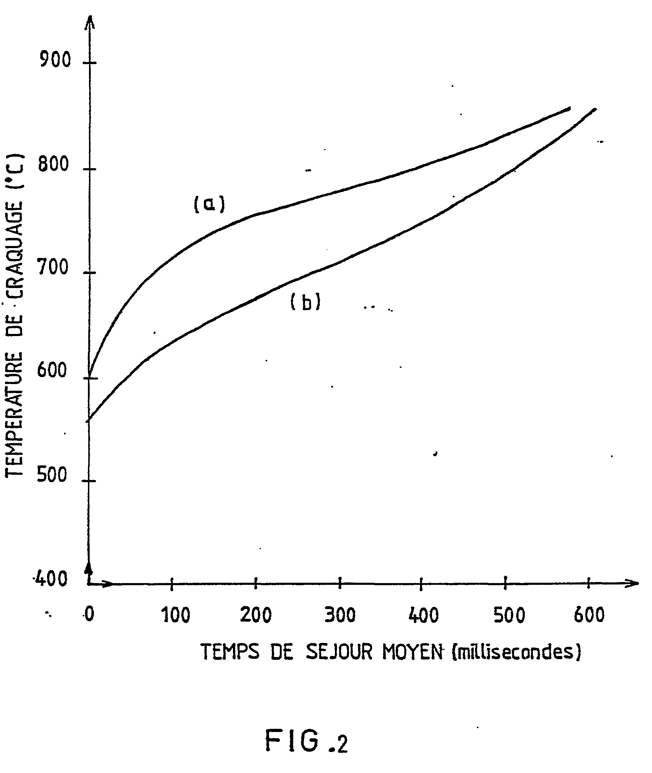

La figure 2 est un graphique représentant l'augmentation de la température de craquage du mélange d'hydrocarbures et de vapeur d'eau circulant dans un tube de craquage depuis l'entrée jusqu'à la sortie de la zone de radiation d'un four de vapocraquage en fonction du temps de séjour moyen du mélange circulant dans ce tube.

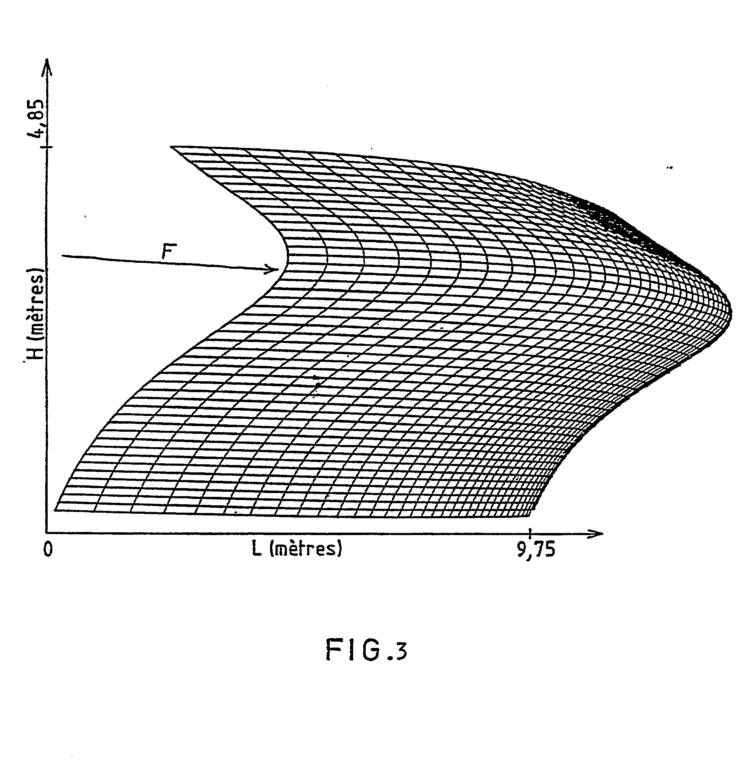

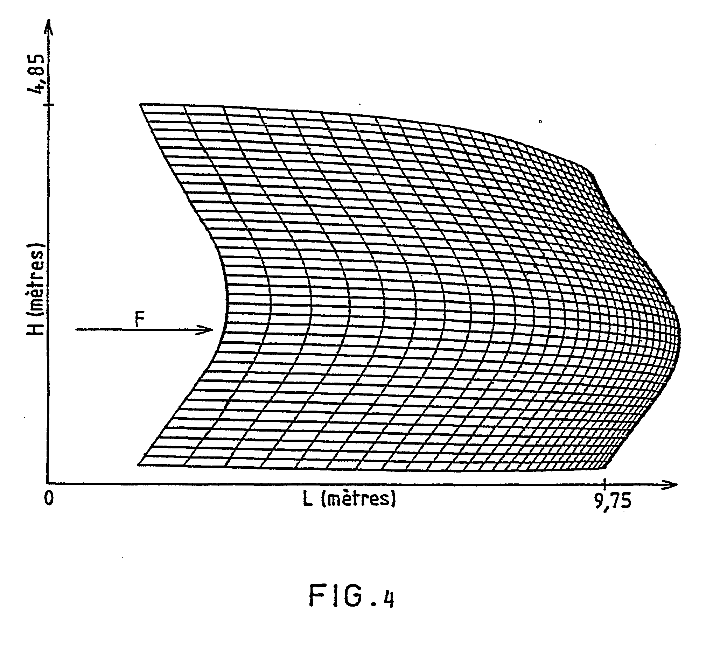

Les figures 3 et 4 sont des graphiques tridimensionnels représentant la répartition du flux thermique à l'intérieur de l'enceinte thermique de radiation d'un four horizontal de vapocraquage, répartition obtenue respectivement selon une puissance de chauffe de type non homogène, telle que décrite selon la présente invention, et une puissance de chauffe de type homogène.

Le procédé selon la présente invention est caractérisé, tout d'abord, par l'évolution de la température de craquage du mélange d'hydrocarbures et de vapeur d'eau circulant dans le tube. Cette température augmente le long du tube de craquage, entre l'entrée et la sortie de la zone de radiation du four, c'est-à-dire dans le sens d'écoulement du mélange. En particulier, la température de craquage du mélange d'hydrocarbures et de vapeur d'eau est à l'entrée de la zone de radiation du four comprise entre 500°C et 700°C, de préférence comprise entre 550°C et 660°C ; elle est à la sortie de cette zone comprise entre 800°C et 880°C, de préférence comprise entre 820°C et 860°C. De préférence, le mélange d'hydrocarbures et de vapeur d'eau est, généralement soumis à un préchauffage avant son entrée dans la zone de radiation du four, ce préchauffage pouvant être réalisé par tout moyen connu, notamment dans une zone de chauffage par convection du four.

Par ailleurs, le procédé de l'invention est caractérisé par le fait que l'augmentation de la température de craquage du mélange est considérablement plus élevée dans la première moitié de la longueur du tube, située vers l'entrée de la zone de radiation du four que dans la deuxième moitié de la longueur du tube située vers la sortie de cette zone. Le rèlage de la température de craquage du mélange d'hydrocarbures et de vapeur d'eau, circulant dans le tube entre l'entrée et la sortie de la zone de radiation du four, est obtenue par une répartition non homogène de la puissance thermique appliquée au tube. En particulier, la puissance thermique appliquée à la première moitié de la lonqueur du tube, située vers l'entrée de la zone de radiation du four, est de 1,5 à 5 fois supérieure, de préférence de 2 à 4 fois supérieure à celle appliquée à la deuxième moitié de la longueur du tube, située vers la sortie de cette zone ; notamment lorsque des hydrocarbures gazeux sont mis en oeuvre dans le procédé. Elle peut être de 1,5 à 4 fois supérieure, de préférence de 2 à 3 fois supérieure à celle appliquée à la deuxième moitié de la longueur du tube, lorsque dans le procédé on met en oeuvre notamment des hydrocarbures liquides. Par puissance thermique, on entend ici la quantité de chaleur apportée par unité de temps et par unité de volume du four environnant le tube de craquage.

Le temps de séjour moyen du mélange d'hydrocarbures et de vapeur d'eau, circulant dans le tube de craquage entre l'entrée et la sortie de la zone de radiation du four, est généralement compris entre 300 et 1800 millisecondes, de préférence compris entre 400 et 1400 millisecondes, notamment lorsqu'on utilise des hydrocarbures gazeux. Il est généralement compris entre 300 et 1000 millisecondes, de préférence compris entre 400 et 800 millisecondes, dans le cas d'hydrocarbures liquides.

Le procédé de l'invention est, également, caractérisé par le volume réactionnel du tube de craquage qui n'est pas identique dans la première et la deuxième moitié de la longueur du tube. Plus précisément, le volume réactionnel de la deuxième moitié de la longueur du tube de craquage, située vers la sortie de la zone de radiation, est de 1,3 à 4 fois supérieur, de préférence de 1,5 à 2,5 fois supérieur à celui de la première moitié de la longueur du tube, située vers l'entrée de cette zone. Par ailleurs, le volume réactionnel par unité de longueur du tube de craquage augmente d'une façon continue ou discontinue depuis l'entrée jusqu'à la sortie de la zone de radiation du four. En pratique, on préfère réaliser cette augmentation d'une façon discontinue, c'est-à-dire par paliers le long du tube de craquage.

On constate que dans ces conditions, la combinaison d'une répartition non homogène de la puissance thermique du four de radiation appliquée le long du tube de craquage avec un volume réactionnel croissant par unité de longueur du tube de craquage a pour résultat d'accroître notablement le temps de séjour moyen du mélange circulant dans la deuxième moitié de la longueur du tube de craquage.

L'effet de cette combinaison permet au mélange d'hydrocarbures et de vapeur d'eau de traverser relativement rapidement la partie du tube de craquage où les température de craquage sont les plus faibles et au contraire plus lentement la partie du tube où les températures de craquage sont les plus élevées. D'une façon inattendue, le procédé de l'invention a pour résultat d'accroître non seulement le rendement et la production horaire en éthylène, mais aussi le taux pondéral de conversion des hydrocarbures dans un four ayant une taille donnée et un tube de craquage ayant une température de peau donnée. Ce résultat est, en outre, obtenu avec une augmentation sensible de la capacité maximale de craquage d'un four ayant une taille déterminée.

La composition du mélange d'hydrocarbures et de vapeur d'eau, mise en oeuvre dans le procédé selon l'invention, est telle que le rapport pondéral de la quantité d'hydrocarbures à la quantité de vapeur d'eau est compris entre 1 et 1O, de préférence compris entre 2 et 6.

Les hydrocarbures mis en oeuvre dans le mélange avec la vapeur d'eau peuvent être des hydrocarbures liquides choisis parmi le naphta, constitué d'hydrocarbures comportant environ de 5 à 1O atomes de carbones, les essences légères constituées d'hydrocarbures comportant environ 5 ou 6 atomes de carbone, le gas-oil constitué d'hydrocarbures comportant environ de 8 à 15 atomes de carbone, ainsi que leurs mélanges. Ils peuvent, en outre, être utilisés en mélange avec dex hydrocarbures saturés et insaturés comportant de 3 à 6 atomes de carbone. Ils peuvent être également des hydrocarbures gazeux constitués par des alcanes comportant de 2 à 4 atomes de carbone, ou par leurs mélanges. Ces alcanes peuvent être mis en oeuvre éventuellement en mélange avec des alcènes comportant de 2 à 6 atomes de carbone et/ou de méthane et/ou des alcanes comportant 5 ou 6 atomes de carbone. On peut, en particulier, mettre en oeuvre dans le procédé de l'invention du gaz naturel, du gaz de pétrole liquéfié, également appelé LPG, ou de l'éthane, produit secondaire issu du vapocraquage d'hydrocarbures liquides tels que le naphta ou le gas-oil.

La présente invention concerne, également, un dispositif permettant la mise en oeuvre du procédé de vapocraquage d'hydrocarbures décrit précédemment, en particulier un dispositif constitué par un four de craquage d'hydrocarbures en présence de vapeur d'eau, comprenant une enceinte thermique de radiation munie de moyens de chauffe, enceinte à travers laquelle passe au moins un tube de craquage où circule le mélange de vapeur d'eau et d'hydrocarbures à craquer, dispositif caractérisé en ce que :

(a) le diamètre interne du tube de craquage augmente d'une façon continue ou discontinue depuis l'entrée jusqu'à la sortie de l'enceinte thermique de radiation, de telle sorte que le rapport entre les diamètres internes du tube à la sortie et à l'entrée de cette enceinte est compris entre 1,3 et 3, et

(b) les moyens de chauffe sont constitués par des brûleurs dont la puissance thermique diminue le long du tube de craquage, depuis l'entrée jusqu'à la sortie de l'enceinte thermique de radiation, de telle sorte que le rapport entre la puissance thermique des brûleurs appliquée à la première moitié de la longueur du tube de craquage, située vers l'entrée de l'enceinte thermique de radiation, et celle appliquée à la deuxième moitié de la longueur du tube, située vers la sortie de cette enceinte, est compris entre 6O/4O et 85/15.

Le four de vapocraquage comprend une enceinte thermique de radiation à travers laquelle passe au moins un tube de craquage diposé sous la forme d'un serpentin horizontal ou vertical. Toutefois, le diamètre moyen interne et la longueur du tube doivent rester dans des domaines de valeurs compatibles avec les contraintes mécaniques et thermiques auxquelles sont soumis les matériaux constituant le tube de craquage. En particulier, le diamètre moyen interne du tube de craquage est compris entre 7O mm et 16O mm, de préférence compris entre 8O mm et 15O mm.

Le four de vapocraquage, selon la présente invention, comprend un tube de craquage dont le diamètre interne augmente d'une façon continue ou discontinue entre l'entrée et la sortie de l'enceinte thermique de radiation, c'est-à-dire dans le sens de l'écoulement du mélange d'hydrocarbures et de vapeur d'eau. En particulier, l'augmentation du diamètre interne du tube de craquage est telle que le rapport entre les diamètre internes du tube à la sortie et à entrée de l'enceinte thermique de radiation est compris entre 1,3 et 3, de préférence compris entre 1,6 et 2,2. En pratique, le diamètre interne du tube de craquage à l'entrée de l'enceinte thermique de radiation est compris de préférence entre 6O et 9O mm, et celui à sortie de cette enceinte est compris de préférence entre 11O et 2OO mm. Ces valeurs tiennent compte du fait que l'on veut éviter d'accroître exagérément les pertes de charge du tube de craquage, notamment dans la partie où le diamètre interne du tube est le plus faible. L'augmentation du diamètre peut être continue tout le long du tube de craquage. Cependant, on préfère mettre en oeuvre un tube de craquage constitué d'une succession de tubes de diamètre interne croissant depuis l'entrée jusqu'à la sortie de l'enceinte thermique de radiation du four. L'augmentation du diamètre interne du tube de craquage est, en particulier, telle que le volume réactionnel de la deuxième moitié de la longueur du tube, située vers la sortie de la zone de radiation, est de 1,3 à 4 fois supérieur, de préférence de 1,5 à 2,5 fois supérieur à celui de la première moitié de la longueur du tube, située vers l'entrée de cette zone.

En pratique, le tube de craquage est disposé sous la forme d'un serpentin constitué d'une succession de section droites reliées entre elles par des coudes, ces sections droites ayant des diamètres internes croissants depuis l'entrée jusqu'à la sortie de l'enceinte thermique de radiation.

Une variante peut consister à mettre en oeuvre un tube de craquage qui, après l'entrée dans l'enceinte thermique de radiation du four, est divisé en un faisceau de tubes parallèles dont le diamètre interne peut être constant, et dont le nombre augmente depuis l'entrée jusqu'à la sortie de l'enceinte thermique, de telle sorte que le volume réactionnel constitué par l'ensemble des tubes correspondant à la deuxième moitié de la longueur du tube de craquage est 1,3 à 4 fois supérieur à celui correspondant à la première moitié de la longueur du tube.

La figure 1 illustre schématiquement un four horizontal de vapocraquage comprenant une enceinte thermique de radiation (1) à travers laquelle passe un tube de craquage disposé sous la forme d'un serpentin constitué de huit sections droites horizontales reliées entre elles par des coudes, les sections (2) et (3) ayant un diamètre interne de 81 mm, les sections (4) et (5) un diamètre interne de 99 mm, les sections (6) et (7) un diamètre interne de 117 mm et les sections (8) et (9) un diamètre interne de 135 mm, l'entrée et la sortie du tube de craquage dans l'enceinte thermique de radiation étant respectivement en (1O) et (11).

Le four de vapocraquage, selon la présente invention, comprend une enceinte thermique de radiation munie de moyens de chauffe constitués de brûleurs, disposés par exemple en rangées sur la sole et/ou sur les murs de l'enceinte. La disposition, le réglage et/ou la taille des brûleurs dans l'enceinte thermique sont tels que la puissance thermique est décroissante le long du tube de craquage depuis l'entrée jusqu'à la sortie de l'enceinte. En particulier, le rapport entre la puissance thermique des brûleurs appliquée à la première moitié de la longueur du tube de craquage, située vers l'entrée de l'enceinte, et celle appliquée à la deuxième moitié de la longueur du tube, située vers la sortie de cette enceinte, est compris entre 6O/4O et 85/15, de préférence compris entre 67/33 et 8O/2O, et en particulier compris entre 67/33 et 75/25. Ce profil décroissant de la puissance thermique des brûleurs appliquée le long du tube de craquage peut être facilement obtenu, en réglant d'une façon appropriée le débit d'alimentation en gaz ou en fuel-gas de chacun des brûleurs. Une autre manière consiste à disposer dans l'enceinte thermique des brûleurs de taille et de puissance de chauffe appropriées. Toutefois, la puissance maximum de chauffe doit être telle que la température de peau n'excède pas la limite compatible avec la nature du métal ou de l'alliage constituant le tube de craquage.

Les exemples non limitatifs suivant illustrent la présente invention.

Exemple 1

Un four de vapocraquage, tel que représenté schématiquement à la figure 1, comprend une enceinte thermique de radiation (1) en briquetage constitué par un parallélépipède rectangle dont les dimensions internes sont de 9,75 m pour la longueur, de 1,7O m pour la largeur et de 4,85 m pour la hauteur. Dans l'enceinte (1), on place un tube de craquage en acier réfractaire à base de nickel et de chrome, ayant un diamètre moyen interne de 1O8 mm, une épaisseur de 8 mm et, compte tenu de la capacité de l'enceinte (1), une longueur totale de 8O mètres, comprise entre l'entrée (1O) et la sortie (11). Ce tube de craquage est disposé sous la forme d'un serpentin, comprenant huit sections droites horizontales, d'égale longueur chacune, reliées entre elles par des coudes. Le diamètre interne des sections (2) et (3) situées vers l'entrée de l'enceinte thermique est de 81 mm ; les sections (4) et (5) qui suivent, ont un diamètre interne de 99 mm ; puis les sections (6) et (7) ont un diamètre interne de 117 mm ; le diamètre interne des sections (8) et (9) situées vers la sortie de l'enceinte thermique est de 135 mm.

Par ailleurs, les diamètres internes du tube de craquage à l'entrée (1O) et à la sortie (11) de l'enceinte (1) étant respectivement de 81 mm et de 135 mm, le rapport entre les diamètres internes du tube à la sortie et à l'entrée est donc de 1,7. Par ailleurs, le volume réactionnel de la deuxième moitié de la longueur du tube de craquage, correspondant aux sections droites (6), (7), (8), (9), est 1,95 fois supérieur au volume réactionnel de la première moitié de la longueur du tube de craquage, correspondant aux sections droites (2), (3), (4) et (5).

L'enceinte thermique de radiation du four de vapocraquage est munie de brûleurs disposés sur les murs de l'enceinte, suivant cinq rangées horizontales, situées à égale distance les unes des autres. La puissance thermique totale est répartie entre les cinq rangées de brûleurs de la façon suivante :
- 4O% de la puissance thermique totale sur la première rangée de brûleurs, située dans le haut de l'enceinte, au voisinage de l'entrée du tube de craquage,
- 27% sur la deuxième rangée de brûleurs, située immédiatement en dessous de la première,
- 18% sur la troisième rangée de brûleurs, située immédiatement en dessous de la deuxième,
- 10% sur la quatrième rangée de brûleurs, située immédiatement en dessous de la troisième, et
- 5% sur la cinquième rangée de brûleurs, située immédiatement en dessous de la quatrième, au voisinage de la sortie du tube de craquage. Ainsi, le rapport entre la puissance thermique des brûleurs appliquée à la première moitié du tube, située vers l'entrée de l'enceinte, et celle appliquée à la deuxième moitié du tube, située vers la sortie de cette enceinte, est de 76/24.

La nappe de flux thermique mesurée à l'intérieur de l'enceinte thermique de radiation du four est, dans ces conditions, représentée à la figure 3 par la surface inscrite dans le graphique tridimensionnel reliant par les trois axes de coordonnée, la longueur L de l'enceinte thermique, la hauteur H de cette enceinte et le flux thermique F. La figure 3 montre, en particulier, que le maximum du flux thermique de radiation se situe dans la partie supérieure de l'enceinte thermique, correspondant à la première moitié de la longueur du tube de craquage située vers l'entrée de l'enceinte thermique de radiation.

Dans ce tube de craquage, on fait circuler un mélange d'éthane et de vapeur d'eau. La composition du mélange d'éthane et de vapeur d'eau mise en oeuvre est telle que le rapport pondéral de la quantité d'éthane à la quantité de vapeur d'eau est de 2,25. On introduit ainsi dans le tube de craquage l'éthane suivant un débit de 1800 kg/h et la vapeur d'eau suivant un débit de 800 kg/h.

La température de craquage du mélange d'éthane et de vapeur d'eau s'élève de 695°C à l'entrée de la zone de radiation du four jusqu'à 848°C à la sortie de cette zone. La pression du mélange est à la sortie du four de 170 kPa. Compte tenu de la répartition du flux thermique dans l'enceinte, la puissance thermique appliquée à la première moitié de la longueur de tube de craquage, située vers l'entrée de la zone de radiation, est 3,1 fois supérieure à celle appliquée à la deuxième moitié de la longueur du tube, située vers la sortie de cette zone.

Le temps de séjour moyen du mélange d'éthane et de vapeur d'eau circulant dans le tube de craquage entre l'entrée et la sortie de la zone de radiation du four est de 530 millisecondes.

Dans ces conditions, on produit par heure 1200 kg d'éthylène et le taux pondéral de conversion de la réaction de vapocraquage est de 83,5 %.

Exemple 2 (comparatif)

Un four de vapocraquage comprend une enceinte thermique de radiation, identique en forme et en taille à celle de l'exemple 1. Dans cette enceinte, on place un tube de craquage en acier réfractaire à base de nickel et de chrome, d'un poids total sensiblement identique à celui de l'exemple 1, ayant un diamètre interne de 108 mm, une épaisseur de 8 mm et, compte tenu de la capacité de l'enceinte et des contraintes thermiques du four, une longueur totale de 80 mètres comprise entre l'entrée et la sortie de l'enceinte. Ce tube de craquage est disposé sous la forme d'un serpentin comprenant huit sections droites horizontales, d'égale longueur chacune, reliées entre elles par des coudes. Le diamètre interne de ces sections droites est constant et égal à 108 mm. Ainsi, les diamètres internes du tube à l'entrée et à la sortie de l'enceinte sont identiques. De même, le volume réactionnel de la première moitié de la longueur du tube de craquage, correspondant aux quatre premières sections droites, est identique au volume réactionnel de la deuxième moitié de la longueur du tube de craquage, correspondant aux quatre dernières sections droites.

L'enceinte thermique de radiation du four de vapocraquage est munie de brûleurs disposés sur les murs de l'enceinte, suivant cinq rangées horizontales, situées à égale distance les unes des autres. La puissance thermique de l'ensemble de ces brûleurs est répartie de façon homogène entre ces cinq rangées. Ainsi, le rapport entre la puissance thermique des brûleurs appliquée à la première moitié et celle appliquée à la deuxième moitié de la longueur du tube de craquage est de 50/50.

La nappe de flux thermique mesurée à l'intérieur de l'enceinte thermique de radiation du four est, dans ces conditions, représentée à la figure 4 par la surface inscrite dans le graphique tridimensionnel reliant par les trois axes de coordonnée, la longueur L de l'enceinte thermique, la hauteur H de cette enceinte et le flux thermique F. La figure 4 montre, en particulier, que le maximum du flux thermique de radiation se situe dans la zone relativement centrale de l'enceinte thermique.

Dans ce tube de craquage, on fait circuler un mélange d'éthane et de vapeur d'eau, identique à celui mis en oeuvre à l'exemple 1. On y introduit l'éthane suivant un débit de 1800 kg/h et la vapeur d'eau suivant un débit de 800 kg/h.

La température de craquage du mélange d'éthane et de vapeur d'eau s'élève de 640°C à l'entrée de la zone de radiation du four jusqu'à 848°C à la sortie de cette zone. La pression du mélange est à la sortie du four de 170 kPa. Compte tenu de la répartition du flux thermique dans l'enceinte, la puissance thermique appliquée à la deuxième moitié de la longueur du tube de craquage est identique à celle appliquée à la première moitié de la longueur du tube.

Le temps de séjour moyen du mélange d'éthane et de vapeur d'eau circulant dans le tube de craquage entre l'entrée et la sortie de la zone de radiation du four est de 580 millisecondes.

Dans ces conditions, on produit par heure 1085 kg d'éthylène, ce qui correspond à une production plus faible que celle obtenue à l'exemple 1. En outre, le taux pondéral de conversion de la réaction de vapocraquage est de 75 %, valeur également plus faible qu'à l'exemple 1.

Exemple 3

On opère dans un four de vapocraquage identique à celui de l'exemple 1. On fait circuler dans le tube de craquage de ce four un mélange constitué d'hydrocarbures gazeux comprenant 64 % en poids de n-butane, 25 % en poids d'isobutane, 10 % en poids de butène-1 et 1 % en poids d'isobutène, et de vapeur d'eau. La composition de ce mélange d'hydrocarbures gazeux et de vapeur d'eau mise en oeuvre est telle que le rapport pondéral de la quantité d'hydrocarbures gazeux à celle de vapeur d'eau est de 2,3. On introduit ainsi dans le tube de craquage les hydrocarbures gazeux et la vapeur d'eau suivant des débits respectivement de 2300 kg/h et de 1000 kg/h.

La température de craquage de ce mélange s'élève de 620°C à l'entrée de la zone de radiation du four jusqu'à 836°C à la sortie de cette zone. La pression de ce mélange est à la sortie du four de 185 kPa.

La puissance thermique appliquée à la première moitié de la longueur du tube de craquage est 3,1 fois supérieure, comme à l'exemple 1, à celle appliquée à la deuxième moitié de la longueur du tube. Le temps de séjour moyen de ce mélange circulant dans le tube de craquage est de 580 millisecondes entre l'entrée et la sortie de la zone de radiation du four.

Dans ces conditions, on produit par heure 700 kg d'éthylène et le taux pondéral de conversion de la réaction de vapocraquage est de 98 % en poids.

## Exemple 4 (comparatif)

On opère dans un four de vapocraquage identique à celui de l'exemple 2. On fait circuler dans le tube de craquage de ce four un mélange identique à celui mis en oeuvre à l'exemple 3 et suivant des débits également identiques à ceux de l'exemple 3.

La température de craquage de ce mélange s'élève de 580°C à l'entrée de la zone de radiation du four jusqu'à 836°C à la sortie de cette zone. La pression de ce mélange est à la sortie du four de 185 kPa. La puissance thermique appliquée à la première moitié de la longueur du tube de craquage est identique, comme à l'exemple 2, à celle appliquée à la deuxième moitié de la longueur du tube.

Le temps de séjour moyen de ce mélange circulant dans le tube de craquage est de 635 millisecondes entre l'entrée et la sortie de la zone de radiation du four.

Dans ces conditions, on produit par heure 610 kg d'éthylène, ce qui correspond à une production plus faible que celle obtenue à l'exemple 3. En outre, le taux pondéral de conversion de la réaction de vapocraquage est de 93 %, valeur également plus faible qu'à l'exemple 3.

## Exemple 5

Un four de vapocraquage, de type vertical, comprend une enceinte thermique de radiation en briquetage constitué par un parallélépipède rectangle dont les dimensions internes sont de 9,75 m pour la longueur, de 1,60 m pour la largeur et de 9,60 m pour la hauteur. Dans l'enceinte, on place un tube de craquage en acier réfractaire à base de nickel et de chrome, ayant un diamètre moyen interne de 98,5 mm, une épaisseur de 8 mm et, compte tenu de la capacité de l'enceinte, une longueur totale de 64 mètres. Le rapport entre la longueur et le diamètre moyen interne est de 650. Ce tube de craquage est disposé sous la forme d'un serpentin, comprenant huit sections droites verticales, d'égale longueur chacune, reliées entre elles par des coudes. Le diamètre interne des deux premières sections situées vers l'entrée de l'enceinte thermique est de 76 mm ; les deux sections qui suivent, ont un diamètre interne de 84 mm ; les deux sections suivantes ont un diamètre interne de 104 mm ; et enfin le diamètre interne des deux dernières sections situées vers la sortie de l'enceinte thermique est de 130 mm.

Par ailleurs, les diamètres internes du tube de craquage à l'entrée et à la sortie de l'enceinte thermique de radiation étant respectivement de 76 mm et de 130 mm, le rapport des diamètres internes du tube à la sortie et à l'entrée est donc de 1,7. Par ailleurs, le volume réactionnel de la deuxième moitié de la longueur du tube de craquage, correspondant aux quatre premières sections droites, est 2,16 fois supérieur au volume réactionnel de la première moitié de la longueur du tube de craquage, correspondant aux quatre dernières sections droites.

L'enceinte thermique de radiation du four de vapocraquage est munie de brûleurs disposés sur les murs de l'enceinte, suivant cinq rangées verticales, situées à égale distance les unes des autres. La puissance thermique des brûleurs est répartie le long du tube de craquage, de telle sorte que la première moitié de la longueur du tube, correspondant aux quatre premières sections droites, reçoit 70 % de la puissance thermique totale, tandis que la deuxième moitié de la longueur du tube, correspondant aux quatre dernières sections droites, reçoit 30 % de la puissance thermique totale.

Dans ce tube de craquage, on fait circuler un mélange d'hydrocarbures liquides et de vapeur d'eau. Les hydrocarbures liquides sont constitués par un naphta de densité 0,684, ayant un intervalle de distillation ASTM 45/185°C et des teneurs pondérales de 38,2 % en paraffines linéaires, de 36,9 % en paraffines ramifiées, de 17,1 % en composés cyclaniques et de 7,8 % en composés aromatiques. La composition du mélange de naphta et de vapeur d'eau mise en oeuvre est telle que le rapport pondéral de la quantité de naphta à la quantité de vapeur d'eau est de 2,1. On introduit ainsi dans le tube de craquage le naphta suivant un débit de 2100 kg/h et la vapeur d'eau suivant un débit de 1000 kg/h.

La température de craquage du mélange de naphta et de vapeur d'eau s'élève de 600°C à l'entrée de la zone de radiation du four jusqu'à 846°C à la sortie de cette zone. L'évolution de la température de craquage du mélange le long du tube de craquage est décrite par la courbe (a) de la figure 2 représentant en abscisse le temps de séjour moyen (en millisecondes) du mélange circulant dans le tube de craquage depuis l'entrée jusqu'à la sortie de la zone de radiation du four et en ordonnée la température de craquage (en °C) du mélange. La courbe (a) montre que la température de craquage du mélange augmente dans sa partie initiale relativement rapidement en fonction du temps de séjour moyen. La température maximale de peau le long du tube de craquage est de 1000°C. La pression du mélange à la sortie du four est de 170 kPa. Compte tenu de la répartition du flux thermique dans l'enceinte thermique de radiation, la puissance thermique appliquée à la première moitié de la longueur du tube de craquage, située vers l'entrée de la zone de radiation, est 2,3 fois supérieure à celle appliquée à la deuxième moitié de la longueur du tube, située vers la sortie de cette zone.

Le temps de séjour moyen du mélange de naphta et de vapeur d'eau circulant dans le tube de craquage entre l'entrée et la sortie de la zone de radiation du four est de 560 millisecondes.

Dans ces conditions, on produit par heure 567 kg d'éthylène et le rendement du craquage en éthylène est de 27 %. Par ailleurs, le taux pondéral de conversion du naphta est de 73 %.

Exemple 6 (comparatif)

Un four de vapocraquage de type vertical comprend une enceinte thermique de radiation identique en forme et en taille à celle de l'exemple 5. Dans cette enceinte, on place un tube de craquage en acier réfractaire à base de nickel et de chrome, ayant un diamètre interne de 98,5 mm, une épaisseur de 8 mm et une longueur totale de 64 mètres comprises entre l'entrée et la sortie de l'enceinte. Le rapport entre la longueur et le diamètre interne du tube est de 650. Ce tube de craquage est disposé sous la forme d'un serpentin comprenant huit sections droits verticales, d'égale longueur chacune, reliées entre elles par des coudes. Le diamètre interne de ces sections droites est constant et égal à 98,5 mm. Ainsi, les diamètres internes du tube à l'entrée et à la sortie de l'enceinte sont identiques. De même, le volume réactionnel de la première moitié de la longueur du tube de craquage, correspondant aux quatre premières sections droites, est identique au volume réactionnel de la deuxième moitié de la longueur du tube de craquage, correspondant aux quatre dernières sections droites.

L'enceinte thermique de radiation du four de vapocraquage est munie de brûleurs disposés sur les murs de l'enceinte. La puissance thermique de l'ensemble de ces brûleurs est répartie de façon homogène le long du tube de craquage, de telle sorte que le rapport entre la puissance thermique des brûleurs appliquée à la première moitié et celle appliquée à la deuxième moitié de la longueur du tube de craquage est de 50/50.

Dans ce tube de craquage, on fait circuler un mélange de naphta et de vapeur d'eau, identique à celui mis en oeuvre à l'exemple 5. On y introduit le naphta suivant un débit de 2100 kg/h et la vapeur d'eau suivant un débit de 1000 kg/h.

La température de craquage du mélange de naphta et de vapeur d'eau s'élève de 560°C à l'entrée de la zone de radiation du four jusqu'à 846°C à la sortie de cette zone. L'évolution de la température de craquage du mélange le long du tube est décrite par la courbe (b) de la figure 2. La courbe (b) montre que la température de craquage du mélange augmente dans sa partie initiale relativement lentement en fonction du temps de séjour moyen. La température de peau maximale le long du tube de craquage est de 1020°C. La pression du mélange à la sortie du four est de 170 kPa.

Le temps de séjour moyen du mélange de naphta et de vapeur d'eau circulant dans le tube de craquage entre l'entrée et la sortie de la zone de radiation du four est de 610 millisecondes.

Dans ces conditions, on produit par heur 504 kg d'éthylène et le rendement du craquage en éthylène est de 24 %. Par ailleurs, le taux pondéral de conversion du naphta est de 70,4 %.

**Revendications**

1. Procédé de fabrication d'oléfines et de dioléfines par craquage d'hydrocarbures liquides ou gazeux en présence de vapeur d'eau, consistant à faire passer, à travers une zone de radiation d'un four, un mélange d'hydrocarbures et de vapeur d'eau circulant dans un tube de craquage placé à l'intérieur de cette zone, en un temps de séjour moyen compris entre 300 et 1800 millisecondes, sous une pression de sortie de four comprise entre 120 et 240 kPa, procédé caractérisé en ce que

(a) la température de craquage du mélange d'hydrocarbures et de vapeur d'eau augmente depuis la température d'entrée de la zone de radiation comprise entre 500 et 700°C jusqu'à la température de sortie de cette zone comprise entre 800 et 880°C, cette augmentation de température étant associée à une répartition non homogène de la puissance thermique du four appliquée le long du tube de craquage, répartition telle que la puissance thermique appliquée à la première moitié de la longueur du tube, située vers l'entrée de la zone de radiation, est de 1,5 à 5 fois supérieure à celle appliquée à la deuxième moitié de la longueur du tube, située vers la sortie de cette zone, et

(b) le volume réactionnel de la deuxième moitié de la longueur du tube de craquage, située vers la sortie de la zone de radiation, est de 1,3 à 4 fois supérieur à celui de la première moitié de la longueur du tube, située vers l'entrée de cette zone.

2. Procédé selon la revendication 1, caractérisé en ce que la puissance thermique appliquée à la première moitié de la longueur du tube est de 1,5 à 4 fois supérieure à celle appliquée à la deuxième moitié de la longueur du tube.

3. Procédé selon la revendication 1, caractérisé en ce que le volume réactionnel de la deuxième moitié de la longueur du tube de craquage est de 1,5 à 2,5 fois supérieur à celui de la première moitié de la longueur du tube.

4. Procédé selon la revendication 1, caractérisé en ce que les hydrocarbures mis en oeuvre sont des hydrocarbures liquides choisis parmi le naphta, les essences légères , le gas-oil, ainsi que leurs mélanges avec les hydrocarbures saturés et insaturés comportant de 3 à 6 atomes de carbone, ou des hydrocarbures gazeux constitués par des alcanes comportant de 2 à 4 atomes de carbone, ou par leurs mélanges, ces alcanes étant éventuellement mis en oeuvre en mélange avec des alcènes comportant de 2 à 6 atomes de carbone et/ou du méthane et/ou des alcanes comportant 5 ou 6 atomes de carbone.

5. Procédé selon la revendication 1, caractérisé en ce que la composition du mélange d'hydrocarbures et de vapeur d'eau mise en oeuvre est telle que le rapport pondéral de la quantité d'hydrocarbures à la quantité de vapeur d'eau est compris entre 1 et 10.

6. Dispositif constitué par un four de craquage d'hydrocarbures en présence de vapeur d'eau, comprenant une enceinte thermique de radiation munie de moyens de chauffe, enceinte à travers laquelle passe au moins un tube de craquage où circule le mélange de vapeur d'eau et d'hydrocarbures à craquer, dispositif caractérisé en ce que

(a) le diamètre interne du tube de craquage augmente d'une façon continue ou discontinue depuis l'entrée jusqu'à la sortie de l'enceinte thermique de radiation, de telle sorte que le rapport entre les diamètres internes du tube à la sortie et à l'entrée de cette enceinte est compris entre 1,3 et 3, et

(b) les moyens de chauffe sont constitués par des brûleurs dont la puissance thermique diminue le long du tube de craquage, depuis l'entrée jusqu'à la sortie de l'enceinte thermique de radiation, de telle sorte que le rapport entre la puissance thermique des brûleurs appliquée à la première moitié de la longueur du tube de craquage, située vers l'entrée de l'enceinte thermique de radiation, et celle appliquée à la deuxième moitié de la longueur du tube, située vers la sortie de cette enceinte, est compris entre 60/40 et 85/15.

7. Dispositif selon la revendication 6, caractérisé en ce que le tube de craquage est constitué d'une succession de tubes de diamètre interne croissant depuis l'entrée jusqu'à la sortie de l'enceinte thermique de radiation.

8. Dispositif selon la revendication 6, caractérisé en ce que le rapport entre la puissance thermique des brûleurs appliquée à la première moitié de la longueur du tube de craquage, située vers l'entrée de l'enceinte thermique de radiation, et celle appliquée à la deuxième moitié de la longueur du tube, située vers la sortie de cette enceinte, est compris entre 67/33 et 80/20.

9. Dispositif selon la revendication 6, caractérisé en ce que le rapport entre les diamètres internes du tube de craquage à la sortie et à l'entrée de l'enceinte thermique de radiation est compris entre 1,6 et 2,2.

10. Dispositif selon la revendication 6, caractérisé en ce que le diamètre interne du tube de craquage à l'entrée de l'enceinte thermique de radiation est compris entre 60 et 90 mm et celui à la sortie de cette enceinte est compris entre 110 et 200 mm.

## Claims

1. A process for the preparation of olefins and diolefins by cracking liquid or gaseous hydrocarbons in the presence of steam, wherein through a radiation zone of a furnace a mixture of hydrocarbons and steam, circulating in a cracking tube disposed within said zone, is caused to flow at a mean residence period in the range of 300 to 1,800 milliseconds and at a furnace outlet pressure in the range of 120 to 240 kPa, characterized in that

(a) the cracking temperature of the mixture of hydrocarbons and steam rises from the inlet temperature of the radiation zone ranging from 500 to 700 °C to the outlet temperature of said zone ranging from 800 to 880 °C, which temperature increase is connected with a non-homogenous distribution of the thermal capacity of the furnace effective alongside the cracking tube, which distribution is such that the thermal capacity effective on the first half of the tube length closer to the inlet of the radiation zone is 1.5 to 5 times greater than that effective on the second half of the tube length closer to the outlet of said zone, and

(b) the reaction volume of the second half of the cracking tube length closer to the outlet of the radiation zone is 1.3 to 4 times greater than that of the first half of the tube length which is located closer to the inlet of said zone.

2. The process according to claim 1, characterized in that the thermal capacity effective on the first half of the tube length is 1.5 to 4 times greater than that effective on the second half of the tube length.

3. The process according to claim 13, characterized in that the reaction volume of the second half of the cracking tube length is 1.5 to 2.5 times greater than that of the first half of the tube length.

4. The process according to claim 1, characterized in that the hydrocarbons used are liquid hydrocarbons selected among naphta, the light petrols, the gas oil as well as among their mixtures with the saturated and unsaturated hydrocarbons having 3 to 6 carbon atoms, or are gaseous hydrocarbons consisting of alkanes having 2 to 4 carbon atoms or of the mixtures thereof, which alkanes optionally are employed as a mixture with alkenes having 2 to 6 carbon atoms and/or methane and/or alkanes having 5 or 6 carbon atoms.

5. The process according to claim 1, characterized in that the composition of the mixture of hydrocarbons and steam employed is such that the weight ratio of the amount of hydrocarbons to the amount of steam is in the range of 1 to 10.

6. An apparatus composed of a furnace for the cracking of hydrocarbons in the presence of steam, which furnace comprises a thermal radiation container equipped with heating means, through which container at least one cracking tube extends in which the mixture of steam and hydrocarbons to be cracked is circulating, characterized in that

(a) the inner diameter of the cracking tube increases in a continuous or non-continuous manner from the inlet to the outlet of the thermal radiation container such that the ratio between the inner diameters of the tube at the outlet and at the inlet of the thermal radiation container is in the range of 1.3 to 3, and

(b) the heating means consist of burners the thermal capacity of which alongside the cracking tube from the inlet to the outlet of the thermal radiation container decreases such that the ratio of the thermal capacity effective on the first half of the cracking tube length closer to the inlet of the thermal radiation container to that effective on the second half of the tube length closer to the outlet of said container ranges from 60/40 to 85/15.

7. The apparatus according to claim 6, characterized in that the cracking tube is composed of a sequence of tubes having inner diameters which are increasing from the inlet towards the outlet of said radiation container.

8. The apparatus according to claim 6, characterized in that the ratio of the thermal capacity of the burners effective on the first half of the cracking tube length closer to the inlet of the thermal radiation container to that effective on the second half of the tube length closer to the outlet of said container ranges from 67/33 to 80/20.

9. The apparatus according to claim 6, characterized in that the ratio between the inner diameters of the cracking tube at the outlet and at the inlet of the thermal radiation container ranges from 1.6 to 2.2.

10. The apparatus according to claim 6, characterized in that the inner diameter of the cracking tube ranges from 60 to 90 mm at the inlet of the thermal radiation container and from 110 to 200 mm at the outlet of said container.

## Patentansprüche

1. Verfahren zur Herstellung von Olefinen und Diolefinen durch Kracken von flüssigen oder gasförmigen Kohlenwasserstoffen in Gegenwart von Wasserdampf, das darin besteht, durch eine Strahlungszone eines Ofens ein Gemisch von Kohlenwasserstoffen und Wasserdampf, das in einem im Inneren dieser Zone angeordneten Krackrohr zirkuliert, bei einer mittleren Verweilsdauer im Bereich von 300 bis 1800 Millisekunden bei einem Ofenausgangsdruck im Bereich von 120 bis 240 kPa strömen zu lassen, dadurch gekennzeichnet, daß

(a) die Kracktemperatur des Gemisches von Kohlenwasserstoffen und Wasserdampf von der Eingangstemperatur der Strahlungszone im Bereich von 500 bis 700 °C bis zur Ausgangstemperatur dieser Zone im Bereich von 800 bis 880 °C steigt, welche Temperatursteigerung mit einer nicht homogenen Verteilung der längs des Krackrohres einwirkenden Wärmeleistung des Ofens verknüpft ist, welche Verteilung derart ist, daß die auf die erste, zum Eingang der Strahlungszone liegende Hälfte der Länge des Rohres einwirkende Wärmeleistung 1,5- bis 5-mal größer als die auf die zweite, zum Ausgang dieser Zone liegende Hälfte der Länge des Rohres einwirkende ist, und

(b) das Reaktionsvolumen der zweiten, zum Ausgang der Strahlungszone liegenden Hälfte der Länge des Krackrohres 1,3- bis 4-mal größer als das der ersten, zum Eingang dieser Zone liegenden Hälfte der Länge des Rohres ist.

2. Verfahren nach dem Anspruch 1, dadurch gekennzeichnet, daß die auf die erste Hälfte der Länge des Rohres einwirkende Wärmeleistung 1,5- bis 4-mal größer als die auf die zweite Hälfte der Länge des Rohres einwirkende ist.

3. Verfahren nach dem Anspruch 1, dadurch gekennzeichnet, daß das Reaktionsvolumen der zweiten Hälfte der Länge des Krackrohres 1,5- bis 2,5-mal größer als das der ersten Hälfte der Länge des Rohres ist.

4. Verfahren nach dem Anspruch 1, dadurch gekennzeichnet, daß die eingesetzten Kohlenwasserstoffe flüssige Kohlenwasserstoffe, die unter dem Naphta, den Leichtbenzinen, dem Gasöl sowie ihren Mischungen mit den gesättigten und ungesättigten Kohlenwasserstoffen mit 3 bis 6 Kohlenstoffatomen gewählt sind, oder gasförmige Kohlenwasserstoffe sind, die aus Alkanen mit 2 bis 4 Kohlenstoffatomen oder aus ihren Mischungen bestehen, welche Alkane eventuell als Mischung mit Alkenen mit 2 bis 6 Kohlenstoffatomen und/oder Methan und/oder Alkanen mit 5 oder 6 Kohlenstoffatomen eingesetzt werden.

5. Verfahren nach dem Anspruch 1, dadurch gekennzeichnet, daß die Zusammensetzung des eingesetzten Gemisches von Kohlenwasserstoffen und Wasserdampf derart ist, daß das Gewichtsverhältnis der Kohlenwasserstoffmenge zur Wasserdampfmenge im Bereich von 1 bis 10 ist.

6. Vorrichtung, die aus einem Ofen zum Kracken von Kohlenwasserstoffen in Gegenwart von Wasserdampf gebildet ist, der einen mit Heizmitteln ausgestatteten Wärmestrahlungsbehälter aufweist, den wenigstens ein Krackrohr durchsetzt, in dem das Gemisch von Wasserdampf und zu krackenden Kohlenwasserstoffen zirkuliert, dadurch gekennzeichnet, daß

(a) der Innendurchmesser des Krackrohres in einer kontinuierlichen oder diskontinuierlichen Weise vom Eingang bis zum Ausgang des Wärmestrahlungsbehälters derart zunimmt, daß das Verhältnis zwischen den Innendurchmessern des Rohres am Ausgang und am Eingang des Wärmestrahlungsbehälters im Bereich von 1,3 bis 3 ist, und

(b) die Heizmittel aus Brennern bestehen, deren Wärmeleistung längs des Krackrohres vom Eingang bis zum Ausgang des Wärmestrahlungsbehälters derart abnimmt, daß das Verhältnis zwischen der auf die erste, zum Eingang des Wärmestrahlungsbehälters liegende Hälfte der Länge des Krackrohres einwirkenden Wärmeleistung und der auf die zweite, zum Ausgang dieses Behälters liegende Hälfte der Länge des Rohres einwirkenden im Bereich von 60/40 bis 85/15 liegt.

7. Vorrichtung nach dem Anspruch 6, dadurch gekennzeichnet, daß das Krackrohr aus einer Folge von Rohren mit vom Eingang bis zum Ausgang des Wärmestrahlungsbehälters zunehmendem Innendurchmesser gebildet ist.

8. Vorrichtung nach dem Anspruch 6, dadurch gekennzeichnet, daß das Verhältnis zwischen der auf die erste, zum Eingang des Wärmestrahlungsbehälters liegende Hälfte der Länge des Krackrohres einwirkenden Wärmeleistung der Brenner und der auf die zweite, zum Ausgang dieses Behälters liegende Hälfte der Länge des Rohres einwirkenden im Bereich von 67/33 bis 80/20 liegt.

9. Vorrichtung nach dem Anspruch 6, dadurch gekennzeichnet, daß das Verhältnis zwischen den Innendurchmessern des Krackrohres am Ausgang und am Eingang des Wärmestrahlungsbehälters im Bereich von 1,6 bis 2,2 ist.

10. Vorrichtung nach dem Anspruch 6, dadurch gekennzeichnet, daß der Innendurchmesser des Krackrohres am Eingang des Wärmestrahlungsbehälters im Bereich von 60 bis 90 mm und der am Ausgang dieses Behälters im Bereich von 110 bis 200 mm ist.

FIG.1

EP 0 252 356 B1

FIG.2

FIG.3

FIG.4